# EUROPEAN PATENT SPECIFICATION

(11) **EP 1 427 471 B1**
(45) Date of publication and mention of the grant of the patent: **27.02.2008**
(21) Application number: 02768788.8
(22) Date of filing: 04.09.2002
(51) Int. Cl.: A61M 37/00

(54) **PLUNGER FOR PATIENT INFUSION DEVICE**
KOLBEN FÜR EINE INFUSIONSVORRICHTUNG FÜR PATIENTEN
PISTON POUR DISPOSITIF DE PERFUSION D'UN PATIENT

(30) Priority: 19.09.2001 US 955623
(43) Date of publication of application: 16.06.2004
(73) Proprietor: Insulet Corporation, Beverly, MA 01915-6120 (US)
(72) Inventor: FLAHERTY, J., Christopher, Topsfield, MA 01983 (US)
(74) Representative: Hill, Justin John
(86) International application number: PCT/US2002/028053
(87) International publication number: WO 2003/024504

(56) References cited:
- WO-A-94/15660
- US-A- 3 631 847
- US-A- 3 812 843
- US-A- 5 800 397
- US-A- 5 993 423

## Description

### Cross-Reference to Related Applications

The present application is related to U.S. patent application serial number 09/943,992, filed on August 31, 2001, which is assigned to the assignee of the present application and incorporated herein by reference.

### Field of the Invention

The present invention relates generally to medical devices, systems and methods, and more particularly to small, low cost, portable infusion devices and methods that are useable to achieve precise, sophisticated, and programmable flow patterns for the delivery of therapeutic liquids to a mammalian patient.

### Background of the Invention

Today, there are numerous diseases and other physical ailments that are treated by various medicines including pharmaceuticals, nutritional formulas, biologically derived or active agents, hormonal and gene based material and other substances in both solid or liquid form. In the delivery of these medicines, it is often desirable to bypass the digestive system of a mammalian patient to avoid degradation of the active ingredients caused by the catalytic enzymes in the digestive tract and liver. Delivery of a medicine other than by way of the intestines is known as parenteral delivery. Parenteral delivery of various drugs in liquid form is often desired to enhance the effect of the substance being delivered, insuring that the unaltered medicine reaches its intended site at a significant concentration. Also, undesired side effects associated with other routes of delivery, such as systemic toxicity, can potentially be avoided.

Often, a medicine may only be available in a liquid form, or the liquid version may have desirable characteristics that cannot be achieved with solid or pill form. Delivery of liquid medicines may best be accomplished by infusing directly into the cardiovascular system via veins or arteries, into the subcutaneous tissue or directly into organs, tumors, cavities, bones or other site specific locations within the body.

Parenteral delivery of liquid medicines into the body is often accomplished by administering bolus injections using a needle and reservoir, or continuously by gravity driven dispensers or transdermal patch technologies. Bolus injections often imperfectly match the clinical needs of the patient, and usually require larger individual doses than are desired at the specific time they are given. Continuous delivery of medicine through gravity feed systems compromise the patient's mobility and lifestyle, and limit the therapy to simplistic flow rates and profiles. Transdermal patches have special requirements of the medicine being delivered, particularly as it relates to the molecular structure, and similar to gravity feed systems, the control of the drug administration is severely limited.

Ambulatory infusion pumps have been developed for delivering liquid medicaments to a patient. These infusion devices have the ability to offer sophisticated fluid delivery profiles accomplishing bolus requirements, continuous infusion and variable flow rate delivery. These infusion capabilities usually result in better efficacy of the drug and therapy and less toxicity to the patient's system. An example of a use of an ambulatory infusion pump is for the delivery of insulin for the treatment of diabetes mellitus. These pumps can deliver insulin on a continuous basal basis as well as a bolus basis as is disclosed in U.S. Patent 4,498,843 to Schneider et al.
A device for delivering fluid to a patient as described in the preamble of claim 1 is known from document WO 94/15 660 A.

The ambulatory pumps often work with a reservoir to contain the liquid medicine, such as a cartridge or reservoir, and use electro-mechanical pumping or metering technology to deliver the medication to the patient via tubing from the infusion device to a needle that is inserted transcutaneously, or through the skin of the patient. The devices allow control and programming via electromechanical buttons or switches located on the housing of the device, and accessed by the patient or clinician. The devices include visual feedback via text or graphic screens, such as liquid crystal displays known as LCD's, and may include alert or warning lights and audio or vibration signals and alarms. The device can be worn in a harness or pocket or strapped to the body of the patient.

Currently available ambulatory infusion devices are expensive, difficult to program and prepare for infusion, and tend to be bulky, heavy and very fragile. Filling these devices can be difficult and require the patient to carry both the intended medication as well as filling accessories. The devices require specialized care, maintenance, and cleaning to assure proper functionality and safety for their intended long term use. Due to the high cost of existing devices, healthcare providers limit the patient populations approved to use the devices and therapies for which the devices can be used.

Clearly, therefore, there was a need for a programmable and adjustable infusion system that is precise and reliable and can offer clinicians and patients a small, low cost, light weight, simple to use alternative for parenteral delivery of liquid medicines.

In response, the applicant of the present application provided a small, low cost, light weight, easy to use device for delivering liquid medicines to a patient. The device, which is described in detail in co-pending U.S. application serial No. 09/943,992, filed on August 31, 2001, includes an exit port, a dispenser for causing fluid from a reservoir to flow to the exit port, a local processor programmed to cause a flow of fluid to the exit port based on flow instructions from a separate, remote control device, and a wireless receiver connected to the local processor for receiving the flow instructions. To reduce the size, complexity and costs of the device, the device is provided with a housing that is free of user input components, such as a keypad, for providing flow instructions to the local processor.

What is still desired are new and improved dispensers and reservoirs for use with devices for delivering fluid to a patient. Preferably, the dispensers and reservoirs will be simple in design, and inexpensive and easy to manufacture, in order to further reduce the size, complexity and costs of fluid delivery devices, such that the devices lend themselves to being small and disposable in nature.

### Summary of the Invention

In response, the present invention provides a device for delivering fluid to a patient, including an exit port assembly adapted to connect to a transcutaneous patient access tool, a reservoir including a side wall extending towards an outlet connected to the exit port assembly, at least one threaded lead screw received in the reservoir and extending towards the outlet of the reservoir generally parallel with the side wall, and a plunger threadedly received on the lead screw such that rotating one of the lead screw and the plunger moves the plunger within the reservoir. The device also includes a dispenser operatively coupled to one of the lead screw and the plunger for rotating one of the lead screw and the plunger. The lead screw driven plunger reduces the size, complexity and costs of the device so that the device lends itself to being small and disposable in nature.

Another device according to the present invention includes an exit port assembly, a reservoir having a side wall extending towards an outlet connected to the exit port assembly, and a plunger slidingly received within the side wall of the reservoir. The device also includes a shaft extending from the plunger and a dispenser operatively coupled to the shaft for causing movement of the shaft along an axis of the shaft. The shaft is relatively incompressible along the axis of the shaft and is bendable traverse to the axis, such that the shaft can be bent yet still used to move the plunger, such that the length of the device can be reduced.

The present invention provides an additional device for delivering fluid to a patient, including an exit port assembly, a reservoir including an outlet connected to the exit port assembly, a plunger movably received in the reservoir for forcing fluid through the outlet upon moving within the reservoir, and a dispenser for moving the plunger within the reservoir. The device also includes a local processor connected to the dispenser and programmed to cause a flow of fluid to the exit port assembly based on flow instructions, a wireless receiver connected to the local processor for receiving flow instructions from a separate, remote control device and delivering the flow instructions to the local processor, and a housing containing the exit port assembly, the reservoir, the dispenser, the local processor, and the wireless receiver. Preferably, the housing is free of user input components for providing flow instructions to the local processor, in order to reduce the size, complexity and costs of the device so that the device lends itself to being small and disposable in nature.

A further device according to the present invention includes an exit port assembly, a reservoir including an outlet connected to the exit port assembly, a plunger movably received in the reservoir for forcing fluid through the outlet to the exit port assembly upon moving within the reservoir, and a dispenser for moving the plunger within the reservoir. A local processor is connected to the dispenser and programmed to cause a flow of fluid to the exit port assembly based upon flow instructions, and further programmed to provide flow information, and a wireless transmitter is connected to the local processor for transmitting the flow information from the local processor to a separate, remote control device. The device also includes a housing containing the exit port assembly, the reservoir, the dispenser, the local processor, and the wireless transmitter, wherein the housing is free of user output components for providing the flow information from the local processor to a user.

These aspects of the invention together with additional features and advantages thereof may best be understood by reference to the following detailed descriptions and examples taken in connection with the accompanying illustrated drawings.

### Brief Description of the Drawings

Fig. 1 is a perspective view of a first exemplary embodiment of a fluid delivery device in accordance with this invention shown secured on a patient, and a remote control device for use with the fluid delivery device (the remote control device being enlarged with respect to the patient and the fluid delivery device for purposes of illustration);

Fig. 2 is a sectional side view of the fluid delivery device of Fig. 1;

Fig. 3 is a sectional side view of a reservoir, a plunger and a lead screw of the fluid delivery device of Fig. 1;

Fig. 4 is an enlarged sectional view of a plunger and lead screw of the fluid delivery device of Fig. 1;

Fig. 5a is a sectional view of the reservoir, the plunger and the lead screw of the fluid delivery device of Fig. 1 taken along line 5--5 of Fig. 3;

Fig. 5b is a sectional view of another exemplary embodiment of a reservoir, a plunger and a lead screw constructed in accordance with the present invention for use with the fluid delivery device of Fig. 1;

Fig. 6 is an exploded sectional side view of another exemplary embodiment of a reservoir, a plunger and a lead screw constructed in accordance with the present invention for use with the fluid delivery device of Fig. 1;

Fig. 7 is a sectional side view of the reservoir, the plunger and the lead screw of Fig. 4;

Fig. 8 is a sectional side view of an additional exemplary embodiment of a reservoir, a plunger and a lead screw constructed in accordance with the present invention for use with the fluid delivery device of Fig. 1;

Fig. 9 is a sectional side view of a further exemplary embodiment of a reservoir, a plunger and a lead screw constructed in accordance with the present invention for use with the fluid delivery device of Fig. 1;

Fig. 10 is a sectional side view of still another exemplary embodiment of a reservoir, a plunger and a lead screw constructed in accordance with the present invention for use with the fluid delivery device of Fig. 1;

Fig. 11 is a sectional side view of an additional exemplary embodiment of a reservoir, a plunger and a lead screw constructed in accordance with the present invention for use with the fluid delivery device of Fig. 1;

Fig. 12 is a sectional side view of a further exemplary embodiment of a reservoir, a plunger and a lead screw constructed in accordance with the present invention for use with the fluid delivery device of Fig. 1;

Fig. 13a is a sectional side view of yet another exemplary embodiment of a reservoir, a plunger and a lead screw constructed in accordance with the present invention for use with the fluid delivery device of Fig. 1;

Fig. 13b is a sectional view of the reservoir, the plunger and the lead screw of Fig. 13a, shown with a needle being inserted into a port of the reservoir;

Fig. 14 is an end elevation view of the plunger of Figs. 13a and 13b;

Fig. 15 is a sectional view of the lead screw and a thread cover of Figs. 13a and 13b;

Fig. 16 is a sectional view of the lead screw and the thread cover coaxially received within the plunger of Figs. 13a and 13b;

Fig. 17a is a side elevation view of the lead screw and the thread cover of Figs. 13a and 13b, wherein threads of the lead screw are covered within the thread cover;

Fig. 17b is a side elevation view of the lead screw and the thread cover of Figs. 13a and 13b, wherein the lead screw has been rotated within the thread cover to reveal the threads of the lead screw;

Fig. 18a is a sectional side view of another exemplary embodiment of a reservoir, a plunger and a lead screw constructed in accordance with the present invention for use with the fluid delivery device of Fig. 1;

Fig. 18b is a sectional view of the reservoir, the plunger and the lead screw of Fig. 18b, shown with a needle being inserted into a port of the reservoir;

Fig. 19 is a sectional side view of an additional exemplary embodiment of a reservoir, a plunger and a lead screw constructed in accordance with the present invention for use with the fluid delivery device of Fig. 1; and

Fig. 20 is a sectional side view of a further exemplary embodiment of a reservoir constructed in accordance with the present invention for use with the fluid delivery device of Fig. 1.

Like reference characters designate identical or corresponding components and units throughout the several views.

### Detailed Description of the Preferred Embodiments

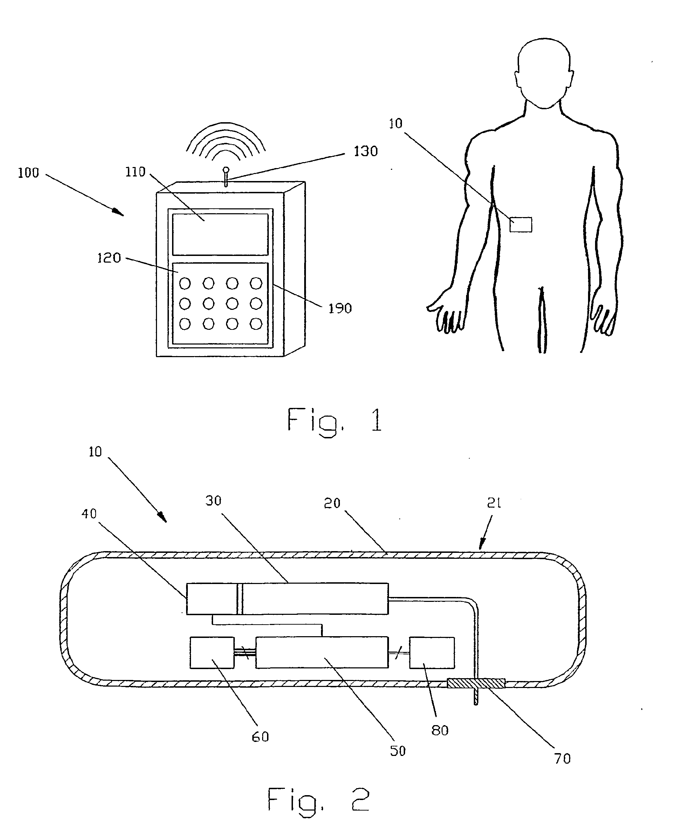

Referring first to Figs. 1 and 2, there is illustrated a fluid delivery device 10 constructed in accordance with the present invention. The types of liquids that can be delivered by the fluid delivery device of the present invention include, but are not limited to, insulin, antibiotics, nutritional fluids, total parenteral nutrition or TPN, analgesics, morphine, hormones or hormonal drugs, gene therapy drugs, anticoagulants, analgesics, cardiovascular medications, AZT or chemotherapeutics. The types of medical conditions that the fluid delivery device of the present invention might be used to treat include, but are not limited to, diabetes, cardiovascular disease, pain, chronic pain, cancer, AIDS, neurological diseases, Alzheimer's Disease, ALS, Hepatitis, Parkinson's Disease or spasticity.

Referring to Fig. 2, the device 10 generally includes an exit port assembly 70 adapted to connect to a transcutaneous patient access tool such as a needle, a dispenser 40 for causing fluid from a reservoir 30 to flow to the exit port assembly, and a processor or electronic microcontroller (hereinafter referred to as the "local" processor) 50 connected to the dispenser.

The local processor 50 is programmed to cause a flow of fluid to the exit port assembly 70 based on flow instructions from a separate, remote control device 100, an example of which is shown in Fig. 1. Referring also to Fig. 2, the fluid delivery device 10 further includes a wireless receiver 60 connected to the local processor 50 for receiving the flow instructions from the separate, remote control device 100 and delivering the flow instructions to the local processor. The device 10 also includes a housing 20 containing the exit port assembly 70, the reservoir 30, the dispenser 40, the local processor 50, and the wireless receiver 60.

As shown, the housing 20 is free of user input components for providing flow instructions to the local processor 50, such as electromechanical switches or buttons on an outer surface 21 of the housing, or interfaces otherwise accessible to a user to adjust the programmed flow rate through the local processor 50. The lack of user input components allows the size, complexity and costs of the device 10 to be substantially reduced so that the device 10 lends itself to being small and disposable in nature.

In order to program, adjust the programming of, or otherwise communicate user inputs to the local processor 50, the fluid delivery device 10 includes the wireless communication element, or receiver 60 for receiving the user inputs from the separate, remote control device 100 of Fig. 1. Signals can be sent via a communication element (not shown) of the remote control device 100, which can include or be connected to an antenna 130, shown in Fig. 2 as being external to the device 100.

The remote control device 100 has user input components, including an array of electromechanical switches, such as the membrane keypad 120 shown. The control device 100 also includes user output components, including a visual display, such as a liquid crystal display (LCD) 110. Alternatively, the control device can be provided with a touch screen for both user input and output. Although not shown in Fig. 1, the remote control device 100 has its own processor (hereinafter referred to as the "remote" processor) connected to the membrane keypad 120 and the LCD 110. The remote processor receives the user inputs from the membrane keypad 120 and provides "flow" instructions for transmission to the fluid delivery device 10, and provides information to the LCD 110. Since the remote control device 100 also includes a visual display 110, the fluid delivery device 10 can be void of an information screen, further reducing the size, complexity and costs of the device 10.

The communication element 60 of the device 10 preferably receives electronic communication from the remote control device 100 using radio frequency or other wireless communication standards and protocols. In a preferred embodiment, the communication element 60 is a two-way communication element, including a receiver and a transmitter, for allowing the fluid delivery device 10 to send information back to the remote control device 100. In such an embodiment, the remote control device 100 also includes an integral communication element 60 comprising a receiver and a transmitter, for allowing the remote control device 100 to receive the information sent by the fluid delivery device 10.

The local processor 50 of the device 10 contains all the computer programs and electronic circuitry needed to allow a user to program the desired flow patterns and adjust the program as necessary. Such circuitry can include one or more microprocessors, digital and analog integrated circuits, resistors, capacitors, transistors and other semiconductors and other electronic components known to those skilled in the art. The local processor 50 also includes programming, electronic circuitry and memory to properly activate the dispenser 40 at the needed time intervals.

In the exemplary embodiment of Fig. 2, the device 10 includes a power supply 80, such as a battery or capacitor, for supplying power to the local processor 50. The power supply 80 is preferably integrated into the fluid delivery device 10, but can be provided as replaceable, e.g., a replaceable battery.

Although not shown, the device can include sensors or transducers such as a reservoir volume transducer or a reservoir pressure transducer, for transmitting information to the local processor 50 to indicate how and when to activate the dispenser 40, or to indicate other parameters determining flow, pump flowpath prime condition, contact sensors, rotary motion or other motion indicators, as well as conditions such as the reservoir 30 being empty or leaking, or the dispensing of too much or too little fluid from the reservoir, etc.

The volume of the reservoir 30 is chosen to best suit the therapeutic application of the fluid delivery device 10 impacted by such factors as available concentrations of medicinal fluids to be delivered, acceptable times between refills or disposal of the fluid delivery device 10, size constraints and other factors. The reservoir 30 may be prefilled by the device manufacturer or a cooperating drug manufacturer, or may include external filling means, such as a fill port.

The exit port assembly 70 can include elements to penetrate the skin of the patient, or can be adapted to connect to a standard infusion device that includes transcutaneous delivery means. A needle connection tubing terminating in a skin penetrating cannula can be provides as an integral part of the exit port assembly 70, for example, with the skin penetrating cannula comprising a rigid member, such as a needle. Alternatively, the exit port assembly 70 can be provided with a Luer connector for connecting to a standard infusion device including a skin penetrating cannula, such as a rigid needle. In any event, the exit port assembly 70 can also be provided with a removable plug (not shown) for preventing leakage during storage and shipment if pre-filled, and during priming if filled by user, and prior to use.

The device 10 can also be provided with an adhesive layer on the outer surface of the housing 20 for securing the device 10 directly to the skin of a patient, as shown in Fig. 1. Although not shown, the adhesive layer is preferably provided in a continuous, oval shape encircling the exit port assembly 70 in order to provide a protective seal around the penetrated skin. The housing 20 can be made from flexible material, or can be provided with flexible hinged sections that allow the fluid delivery device 10 to flex during patient movement to prevent detachment and aid in patient comfort.

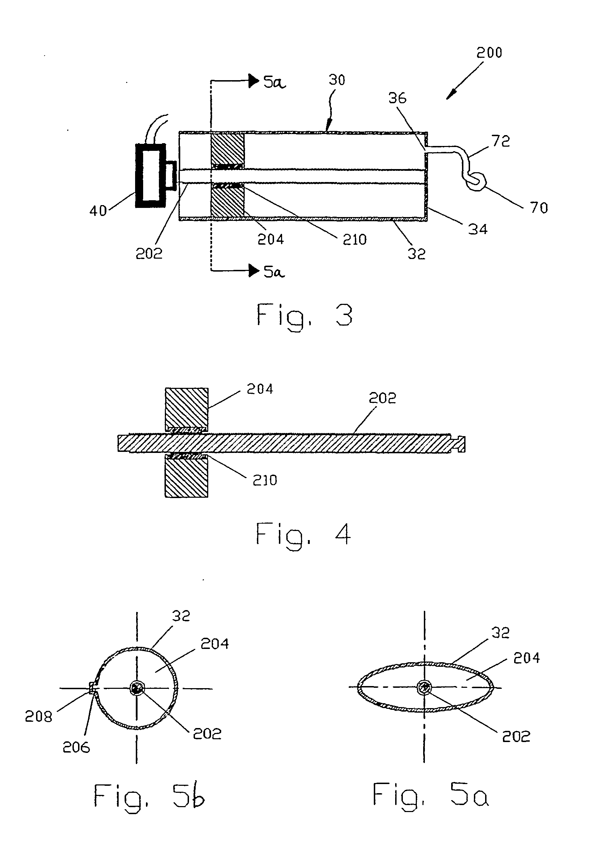

Referring to Figs. 3 through 22, the present disclosure provides various combinations of dispensers 40 and reservoirs 30 for use with the fluid delivery device 10 of Figs. 1 and 2. The dispensers 40 and reservoirs 30 are small and simple in design, and inexpensive and easy to manufacture, in order to further reduce the size, complexity and costs of the fluid delivery device 10, such that the device 10 continues to lend itself to being small and disposable in nature.

Referring to Figs. 3 through 5, a first combination 200 of a reservoir 30 and a dispenser 40 constructed in accordance with the present invention is shown. The reservoir 30 has a side wall 32 extending between an open end and an end wall 34 of the reservoir. The end wall 34 includes an outlet 36 connected through a lumen 72 to the exit port assembly 70 of the device 10.

The reservoir 30 also includes a threaded lead screw 202 mounted for rotation within the reservoir 30, and a plunger 204 threadedly received on the lead screw. The lead screw 202 is positioned coaxial with the side wall 32 and extends to the end wall 34 of the reservoir 30. The plunger 204 and the reservoir 30 are adapted such that a seal is formed between the plunger 204 and the lead screw 202 and the plunger 204 and the side wall 32 of the reservoir, so that movement of the plunger 204 towards the end wall 34 of the reservoir 30 will force fluid through the outlet 36 to the exit port assembly 70.

The plunger 204 is prevented from rotation with respect to the side wall 32 so that, when the screw 202 is turned with respect to the plunger 204, the plunger is caused to move along the screw 202 within the reservoir 30. In a preferred embodiment shown in FIG. 5a, the reservoir 30 and the plunger 204 are provided with corresponding non-circular cross-sections. The cross-sections are oval, but the reservoir 30 and the plunger 204 can be provided with other non-circular cross-sections, such as square or rectangular. In another preferred embodiment shown in FIG. 5b, the reservoir 30 and the plunger 204 are provided with circular cross-sections, but the plunger 204 has at least one protrusion 206 radially extending into a channel 208 in the side wall 32 of the reservoir 30 to prevent rotation of the plunger. The width and the length of the reservoir 30 is chosen to minimize the overall size of the fluid delivery device.

A significant advantage of the reservoir 30 utilizing an integrated lead screw 202 upon which the plunger 204 rides, is the significant length reduction as compared to a standard syringe basically including a reservoir with a separate sliding plunger extending out of the reservoir. Another advantage of the reservoir 30 according to the present invention is that the plunger 204 and the internal lead screw 202 are entirely contained within the reservoir 30, and do not require mechanisms or procedures for pulling the plunger back to remove a used syringe or re-load a full syringe. Such mechanisms or procedures can increase the costs, complexity, and size and weight, and decrease the reliability of a fluid delivery device. Thus, the reservoir 30 of the present invention advantageously does not need such mechanisms or procedures.

In order to further reduce the cost of the reservoir 30, the lead screw 202 and the plunger 204 are preferably made from an inexpensive material. The lead screw 202 is made of a rigid material such as a metal, such as stainless steel, or a plastic, such as polyethylene or polypropylene. The side wall 32 and the end wall 34 of the reservoir are preferably made from a rigid plastic. The plunger 204, however, is made of a flexible material, such as a silicone elastomer or rubber, and provided with a rigid insert 210 made of metal or plastic for engaging the threads of the lead screw 202. Since the device is preferably disposable, preventing thread wear between the lead screw 202 and the plunger 204 is not necessary, thereby allowing the use of less expensive materials and lower tolerances in the manufacture and assembly of the lead screw 202 and the plunger 204.

In order to turn the lead screw 202 of the reservoir 30, the dispenser generally comprises a rotational drive assembly 40. The rotational drive assembly 40 can be configured to provide either continuous flow or pulse volume flow (e.g., less than one microliter for insulin infusion). The specific form of such a rotational drive assembly 40 can include motors, such as stepper motors, dc motors, ac motors, piezo motors, ultrasound motors or other motors; or solenoid or other linear actuators that drive ratcheting gear assemblies; or piezo materials attached to the lead screw 202 and driven with energy, such as electrical, mechanical, sound, chemical, or thermal energy; or magnetic drives. In the embodiment 200 of Fig. 3, the dispenser is provided as an electric motor 40 connected to an end of the lead screw 202 for turning the lead screw upon being activated by the local processor of the device 10.

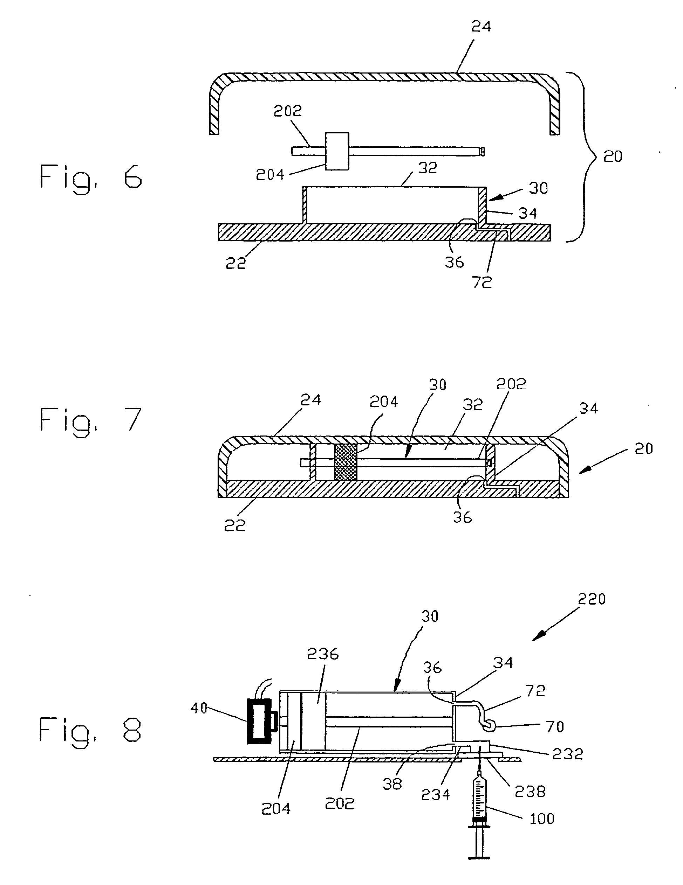

Referring to Figs. 6 and 7, another reservoir 30 constructed in accordance with the present invention is shown. The reservoir 30 is unitarily formed as part of the housing 20 of the fluid delivery device 10, in order to reduce parts and simplify the manufacturing process. The housing 20 includes a base 22 and a cover 24, which are assembled about the lead screw 202 and the plunger 204 of the reservoir 30. In the embodiment shown, for example, the base 22 of the housing 20 defines the end walls 34 of the reservoir 30, while the base 22 and the cover 24 define the side walls 32 of the reservoir 30. In addition, the outlet 36 is formed in the side wall 32 of the reservoir 30.

Referring to Fig. 8, another embodiment 220 of a reservoir 30 and a dispenser 40 constructed in accordance with the present invention is shown. The embodiment 220 is similar to the embodiment 200 of Fig. 3, but further includes an inlet 38 at the end wall 34 of the reservoir 30 connected through a lumen 234 to a fill port 232. The reservoir 30 also includes a second plunger 236 slidingly received on the lead screw 202 between the first plunger 204 and the end wall 34 of the reservoir 30. The second plunger 236 is adapted such that a seal is provided between the second plunger and the lead screw 202 and a seal is provided between the second plunger and the lead screw and the second plunger and the side wall 32 of the reservoir 30. A seal is not necessary around the first plunger 204, but the first plunger is prevented from rotating within the reservoir.

The fill port 232 can include a needle insertion septum 238 for receiving a needle 100, as shown. Needle insertion septum 238 may be constructed of a resealing elastomer such as silicone that allows a needle 100 to puncture the septum 238 to add fluid to the reservoir 30, yet reseal after the needle is withdrawn. Alternatively, the fill port 232 can include a Luer or other connector. Although not shown the exit port assembly 70 can be provided with a plug for preventing leakage from the outlet 36 of the reservoir 30 during filling of the reservoir, or can include other manual or automatic outlet flow path constriction means.

The second plunger 236 is adapted to slide on lead screw 202 towards the first plunger 204 during filling of the reservoir. For partial fills, the second plunger will not be in contact with the first plunger 204. In a priming process, the lead screw 202 can be rotated to cause the first plunger 204 to move up against the second plunger 236. If a fill port is used with the embodiment 200 of Fig. 3 having the single plunger 204, the reservoir 30 and other fluid path components may be placed in a vacuum during the final manufacturing process to simplify filling and priming of the fluid delivery device 10 for the patient. In any event, in the pre-filled position, the plunger 236 is preferably located adjacent to the end wall 34 of the reservoir 30 to minimize air in the fluid path.

Sensors can be provided for monitoring the position of each plunger 204, 236 and indicating when the plungers are in contact, the amount of fluid remaining in reservoir, and whether proper infusion is occurring, for example. The plungers 204, 236 should be in contact upon beginning fluid therapy so that initial rotations of the lead screw 202 will cause fluid to flow, as expected.

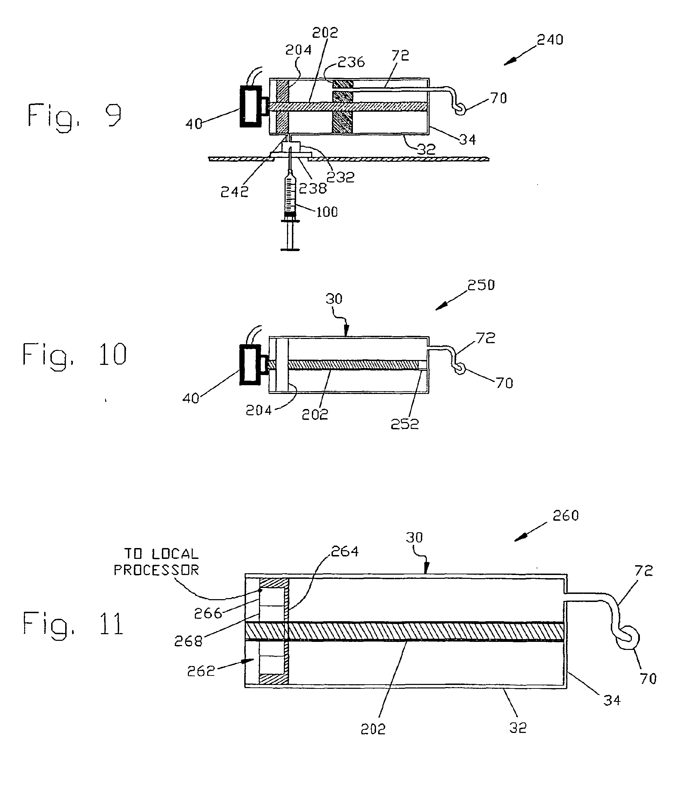

Referring to Fig. 9, an additional embodiment 240 constructed in accordance with the present invention is shown. The embodiment 240 is similar to the embodiment 220 of Fig. 8, but includes an inlet 242 in the side wall 32 of the reservoir 30 connected through a lumen 234 to a fill port 232 of the device 10, and the lumen 72 connected to the exit port assembly 70 extends through the second plunger 236. Initially, the second plunger 236 and the first plunger 204 are on opposite sides of the inlet 242. If the plungers 236, 204 are also initially separated, then a vacuum can be provided between the plungers. If the plungers 236, 204 are initially in contact and aligned with the inlet 242, then a vacuum is not necessary between the plungers.

The second plunger 236 is designed to be moved on the lead screw 202 by fill pressure but not infusion pressure. Upon filling the reservoir 30 through the fill port 232, the second plunger 236 is moved towards the end wall 34 of the reservoir by the fill pressure. Then, during use, the dispenser 40 causes the first plunger 204 to move towards the second plunger 236 and create infusion pressure sufficient to force fluid out of the reservoir 30 to the exit port assembly 70, but not sufficient to move the second plunger. The first plunger 204 eventually passes over the inlet 242 and prevents further filling of the reservoir 30 after infusion has begun.

Referring to Fig. 10, a further embodiment 250 constructed in accordance with the present invention is shown. The embodiment 250 is similar to the embodiment 200 of Fig. 3, but includes a lead screw 202 having a non-threaded portion 252 adjacent to the end wall 34 of the reservoir 30. The non-threaded portion 252 is designed such that the plunger 204 is moved onto the non-threaded portion 252 as the reservoir 30 is emptied of fluid, and the plunger 204 becomes stranded on the non-threaded portion. The non-threaded portion 252 of the lead screw 202, therefore, prevents reuse of the reservoir 30.

Referring to Fig. 11, yet another embodiment 260 constructed in accordance with the present invention is shown. The embodiment 260 is similar to the embodiment 200 of Fig. 3, but includes a dispenser provided in the form of a motor 262 mounted within a plunger 264. The motor 262 includes an outer portion 266 secured to the plunger 264 and an inner portion 268 threadedly engaging the lead screw 202, which is fixed for non-rotation within the reservoir 30. The outer portion 266 turns the inner portion 268 to move the plunger 264 along the lead screw 202. Electrical wires for connection to a local processor of device extend from the non-rotating outer portion 266 of the motor 262 to avoid wires twisting, and the wires are flexible and long enough to follow travel of the plunger 264 during a fill process and an infusion process.

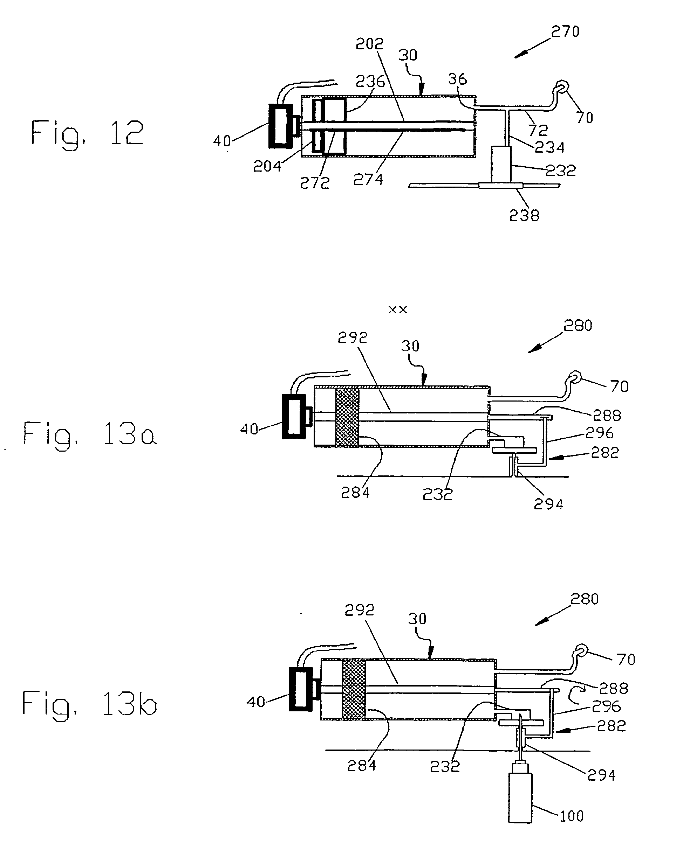

Referring to Fig. 12, another embodiment 270 constructed in accordance with the present invention is shown. The embodiment 270 is similar to the embodiment 220 of Fig. 8 but includes a sensor 272 for determining the position of the plunger 236 within the reservoir 30. Knowing the position of the plunger 236 allows a determination of the volume of fluid remaining in the reservoir 30, such that proper fluid flow can be confirmed. In the embodiment shown, the lead screw 202 includes a linear encoder 274 and the sensor comprises a magnetic sensor 272 mounted on the plunger 236. However, other sensors can alternatively be used for determining the position of the plunger 236 and the volume of fluid contained in the reservoir 30.

In the embodiment 270 of Fig. 12, the inlet lumen 234 is connected to the outlet lumen 72, such that the outlet 36 is used to both fill and empty the reservoir 30. This arrangement maximizes the amount of the outlet lumen 72 and the exit port assembly 70 that is primed with fluid prior to an infusion process.

An additional embodiment 280 constructed in accordance with the present invention is shown in Figs. 13a and 13b. The embodiment 280 is similar to the embodiment 200 of Fig. 3, but includes an inlet 38 at the end wall 34 of the reservoir 30 connected through a lumen 234 to a fill port 232 having a needle insertion septum 238 for receiving a needle 100, as shown. A release mechanism 282 is also provided for disengaging a plunger 284 from a lead screw 288 upon a needle 100 being inserted into the fill port 232.

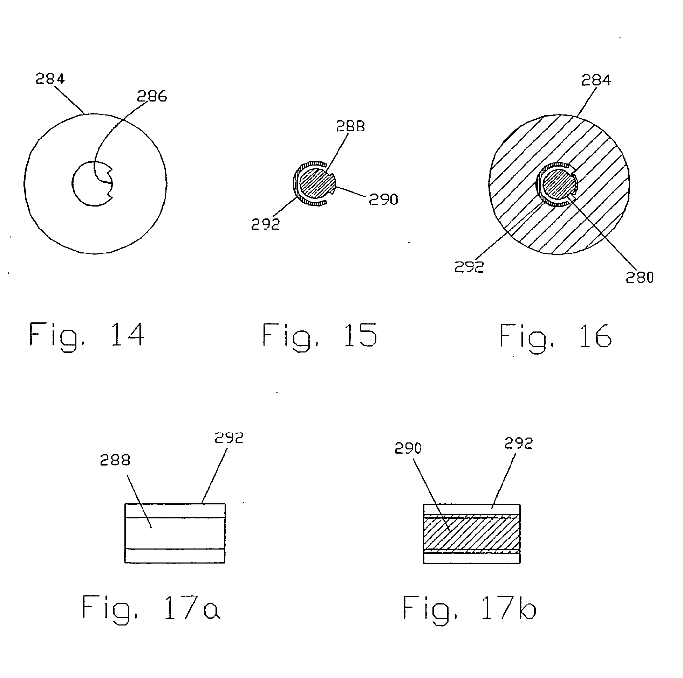

Referring also to Figs. 14 through 16, the plunger 284 has partial threads 286, and the reservoir 30 includes a lead screw 288 having partial threads 290. A thread cover 292 is positioned between the lead screw 288 and the plunger 284 to prevent engagement of the partial threads 290 of the lead screw and the partial threads 286 of the plunger upon a needle 100 being inserted into the fill port 232, such that the plunger can slide upon the thread cover 292 upon the reservoir being filled.

In the embodiment shown, the release mechanism 282 comprises a collar 294 positioned for frictionally receiving a needle 100 entering the fill port 232 and a lever 296 extending from the collar 294 to the lead screw 288. As shown in Fig. 13b, a needle 100 inserted into the fill port 232 moves the collar 294 and the lever 296, which in turn rotates the lead screw 288 with respect to the thread cover 292. As shown in Fig. 17a, rotating the lead screw 288 with respect to the thread cover 292 covers the partial threads 290 of the lead screw, such that the plunger can slide on the thread cover 292 upon the reservoir being filled. Removing the needle 100 from the fill port 232 moves the collar 294 and the lever 296 back, which in turn rotates the lead screw 288 with respect to the thread cover 292 and uncovers the partial threads 290 of the screw 288, such that the screw 288 engages the plunger. Thereafter, rotation of the screw 288 and the thread cover 292 causes the plunger 284 to move within the reservoir 30 and force fluid to the exit port assembly 70.

Thus, the plunger 284 and the lead screw 288 are automatically disengaged during filling, and re-engaged after filling to allow repeated filling. Alternatively, the reservoir 30 can be supplied to a user with the plunger 284 and the lead screw 288 initially disengaged, but re-engaged after filling, to allow only a single filling of the reservoir. In addition, an embodiment can be provided with only the plunger 284 and the lead screw 288, and not the cover 292, wherein the partial threads of the plunger and the lead screw are disengaged upon needle 100 insertion.

An alternative to the disengagement embodiments is the two plunger system, such as the embodiment 220 of Fig. 8, where the first plunger 204 engages the lead screw 202 and the second plunger 236 just seals around the lead screw (and reservoir wall). A single plunger option is also viable, whereby the geometry and materials of construction of the plunger and lead screw threads allow the plunger to move backward during a fill, yet be driven forward by turning the lead screw to infuse fluid. In such an embodiment, the fill pressure would need to be greater than the (driving) infusion pressure.

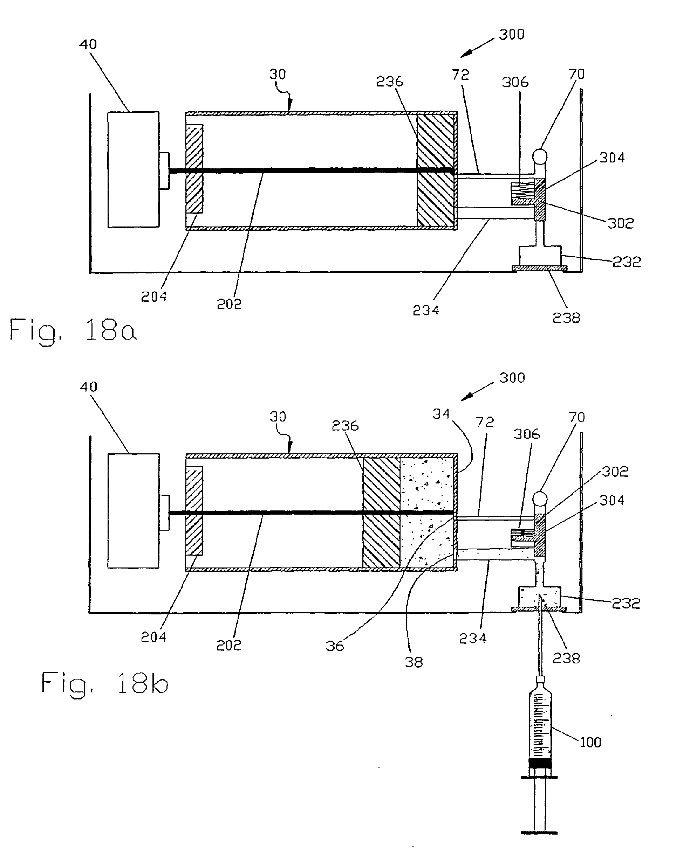

Another embodiment 300 constructed in accordance with the present invention is shown in Figs. 18a and 18b. The embodiment 300 is similar to the embodiment 220 of Fig. 8, but includes a plug 302 for closing the outlet 36 of the reservoir 30 upon the reservoir being filled through the inlet 38. In particular, the embodiment 300 is provided with a passageway 304 connecting the lumen 72 of the outlet 36 and the lumen 234 of the inlet 38, and the plug 302 is movably positioned in the passageway 304. The plug 302 is biased, by a spring 306 for example, to a first position opening the outlet 36 of the reservoir and sealing the inlet 38 of the reservoir, as shown in Fig. 18a, and is movable to a second position sealing the outlet 36 of the reservoir and opening the inlet 38 of the reservoir, as shown in Fig. 18b.

Upon the reservoir 30 being filled through the fill port 232, the pressure of the fluid forces the plug 302 to the second position to seal the outlet 36 of the reservoir and ensure that the fluid does not leak through the exit port assembly 70 as the reservoir 30 is filled and the second plunger 236 is moved by the fluid towards the first plunger 204. The geometry of the plug 302 is chosen such that as the fill process begins, the outlet 36 is closed before the inlet 38 is opened, and as the fill is completed, the inlet 38 is closed before the outlet 36 is opened.

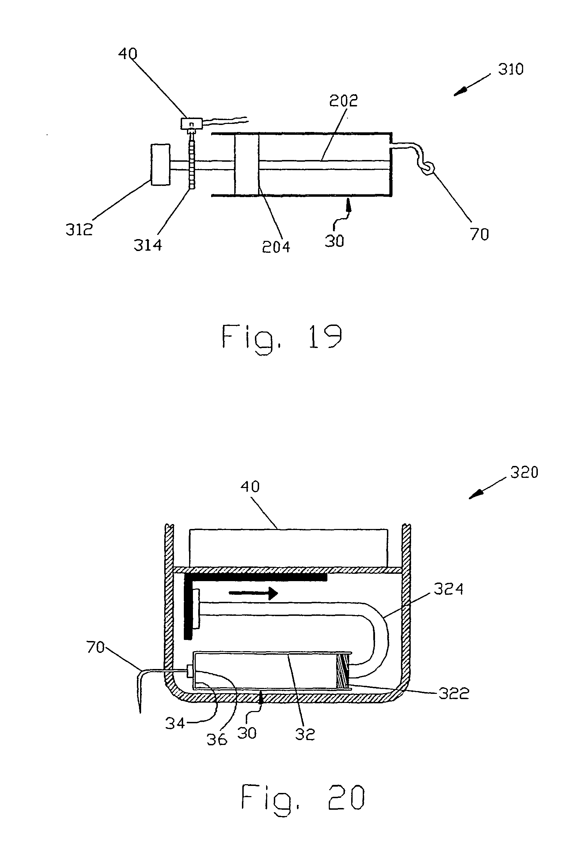

Fig. 19 shows yet another embodiment 310 constructed in accordance with the present invention. The embodiment 310 is similar to the embodiment 200 of Fig. 3, but includes a coiled clock spring 312 for turning the rotatable lead screw 202. A gear 314 radially extends from the lead screw 202, and the dispenser is provided in the form of a ratchet 40 controlled by the local processor. The ratchet 40 is moveable between a position engaging the gear 314 and preventing the clock spring 312 from rotating the lead screw 202, and a position disengaging the gear 314 and allowing the clock spring 312 to rotate the lead screw. Thus, a command from the local processor for the ratchet 40 to disengage the gear 314 causes the plunger 204 to be moved on the threaded lead screw 202 through the reservoir 30.

A further exemplary embodiment 320 constructed in accordance with the present invention is shown in Fig. 20. The embodiment 320 includes a barrel-like reservoir 30 having a tubular side wall 32 extending between an open end and an end wall 34 of the reservoir. The end wall includes an outlet 36 connected to the exit port assembly 70 of the device 10. A plunger 322 is slidingly received within the side wall 32 of the reservoir 30, a shaft 324 extends from the plunger, and a dispenser comprises a rotational drive assembly 40 adapted to linearly move the shaft 324 upon being activated by the local processor of the device 10.

The plunger 322 and the reservoir 30 are adapted such that a seal is formed between the plunger and the side wall 32 of the reservoir, so that movement of the plunger towards the end wall 34 of the reservoir will force fluid through the outlet 36 to the exit port assembly 70. The shaft 324 is flexible such that the reservoir 30 can be "folded" under the drive assembly 40, as shown, to reduce the overall length of the fluid deliver device 10. Although not shown, the housing of the device can be provided with structure for maintaining and guiding the bent shaft 324 as the plunger 322 is advanced in the reservoir 30.

Although exemplary embodiments of the invention have been shown and described, many changes, modifications and substitutions may be made by those having ordinary skill in the art without necessarily departing from the scope of this invention.

## Claims

1. A device (10) for delivering fluid to a patient, comprising:
a) an exit port assembly (70) adapted to connect to a transcutaneous patient access tool ;
b) a reservoir (30) including a side wall (32) extending from a first end wall towards a second end wall (34) and an outlet (36) connected to the exit port assembly (70);
c) at least one threaded lead screw (202) received in the reservoir (30) and extending towards the outlet (36) of the reservoir (30) generally parallel with the side wall (32)
d) a plunger (204) threadedly received on the lead screw (202) such that rotating one of the lead screw (202) and the plunger (204) moves the plunger (204) within the reservoir (30); and
e) a dispenser (40) operatively coupled to one of the lead screw (202) and the plunger (204) for rotating one of the lead screw (202) and the plunger (204), **characterised in that** the lead screw (202) is coupled to the second end wall (34).

2. A device according to Claim 1, wherein the dispenser (40) rotates the lead screw (202).

3. A device according to Claim 2, wherein the plunger (204) is prevented from rotating with respect to the side wall (32) of the reservoir (30).

4. A device according to Claim 1 Claim 2 or Claim 3, wherein the plunger (209) includes an insert (210) threadedly received on the lead screw (202) and wherein the threaded insert (210) and the plunger (204) are made from different materials.

5. A device according to any of Claims 1 to 4, wherein the threaded lead screw (202) is made from a plastic.

6. A device according to any of Claims 1 to 5, wherein the dispenser (40) comprises a motor.

7. A device according to any of Claims 1 to 6, further comprising a transcutaneous patient access tool connected to the exit port assembly (70).

## Patentansprüche

1. Vorrichtung (10) zum Liefern von Fluid an einen Patienten, umfassend:
a) eine Austrittsöffnungsanordnung (70), die angepasst ist, um mit einem Patienten-Transkutanzugriffswerkzeug verbunden zu sein;
b) ein Reservoir bzw. Vorratsbehälter (30), der eine Seitenwand (32), die sich von einer ersten Endwand zu einer zweiten Endwand (34) hin erstreckt, und einen Auslass (36) enthält, der mit der Austrittsoffnungsanordnung (70) verbunden ist;
c) wenigstens eine Gewindeleitspindel (202), die in dem Vorratsbehälter (30) aufgenommen ist und sich zu dem Auslass (36) des Vorratsbehälters (30) im Allgemeinen parallel mit der Seitenwand (32) erstreckt.
d) einen Kolben (204), der gewindemäßig auf bzw. an der Leitspindel (202) aufgenommen ist, so dass ein Drehen der Leitspindel (202) oder des Kolbens (204) den Kolben (204) in dem Vorratsbehälter (30) bewegt; und
e) eine Abgabeeinrichtung (40), die wirkungsmäßig bzw. operativ mit der Leitspindel (202) oder dem Kolben (204) gekoppelt ist, um die Leitspindel (202) oder den Kolben (204) zu drehen,
**dadurch gekennzeichnet, dass** die Leitspindel (202) mit der zweiten Endwand (34) gekoppelt ist.

2. Vorrichtung nach Anspruch 1, wobei die Abgabeeinrichtung (40) die Leitspindel (202) dreht.

3. Vorrichtung nach Anspruch 2, wobei der Kolben (204) daran gehindert wird, sich bezüglich der Seitenwand (32) des Vorratsbehälters (30) zu drehen.

4. Vorrichtung nach Anspruch 1, Anspruch 2 oder Anspruch 3, wobei der Kolben (209) einen Einsatz (210) enthält, der gewindemäßig auf bzw. an der Leitspindel (202) aufgenommen ist, und wobei der Gewindeeinsatz (210) und der Kolben (204) aus unterschiedlichen Materialien bestehen.

5. Vorrichtung nach einem der Ansprüche 1 bis 4, wobei die Gewindeleitspindel (202) aus einem Kunststoff besteht.

6. Vorrichtung nach einem der Ansprüche 1 bis 5, wobei die Abgabeeinrichtung (40) einen Motor umfasst.

7. Vorrichtung nach einem der Ansprüche 1 bis 6, ferner umfassend ein Patienten-Transkutanzugriffswerkzeug, das mit der Austrittsöffnungsanordnung (70) verbunden ist.

## Revendications

1. Dispositif (10) de délivrance d'un fluide à un patient, comprenant :
a) un ensemble formant orifice de sortie (70) adapté pour être relié à un outil d'accès transcutané au patient ;
b) un réservoir (30) comprenant une paroi latérale (32) s'étendant à partir d'une première paroi d'extrémité en direction d'une seconde paroi d'extrémité (34) et une sortie (36) reliée à l'ensemble formant orifice de sortie (70) ;
c) au moins une vis-mère filetée (202) reçue dans le réservoir (30) et s'étendant en direction de la sortie (36) du réservoir (30) généralement parallèle avec la paroi latérale (32) ;
d) un piston (204) reçu par filetage sur la vis-mère (202) de sorte que si l'on tourne l'une des vis-mère (202), le piston (204) déplace le piston (204) dans le réservoir (30) ; et
e) un distributeur (40) couplé de manière opérationnelle à l'une des vis-mère (202) et le piston (204) pour tourner l'une des vis-mère (202) et le piston (204), **caractérisé en ce que** la vis-mère (202) est couplée à la seconde paroi d'extrémité (34).

2. Dispositif selon la revendication 1, dans lequel le distributeur (40) tourne la vis-mère (202).

3. Dispositif selon la revendication 2, dans lequel le piston (204) est empêché de tourner par rapport à la paroi latérale (32) du réservoir (30)

4. Dispositif selon la revendication 1, 2 ou 3, dans lequel le piston (209) comprend un insert (210) reçu par filetage sur la vis-mère (202) et dans lequel l'insert fileté (210) et le piston (204) se composent de matériaux différents.

5. Dispositif selon l'une quelconque des revendications 1 à 4, dans lequel la vis-mère filetée (202) se compose de plastique.

6. Dispositif selon l'une quelconque des revendications 1 à 5, dans lequel le distributeur (40) comprend un moteur.

7. Dispositif selon l'une quelconque des revendications 1 à 6, comprenant en outre un outil d'accès transcutané au patient relié à l'ensemble formant sortie (70).
